# EUROPEAN PATENT APPLICATION

(11) **EP 3 636 230 A1**
(43) Date of publication of application: **15.04.2020**
(21) Application number: 19202244.0
(22) Date of filing: 09.10.2019
(51) Int. Cl.: A61F 9/007, A61F 9/013

(54) **DEVICE FOR PERFORMING SURGICAL MANIPULATION OF THE SCLERA**

(30) Priority: 12.10.2018 US 201862745155 P
(71) Applicant: Kaplan, Eran, 4282300 Zoran (IL); Daas, Kamal, 6158101 Tel Aviv (IL)
(72) Inventor: Kaplan, Eran, 4282300 Zoran (IL); Daas, Kamal, 6158101 Tel Aviv (IL)
(74) Representative: Trinks, Ole

(57) **Abstract**

A device for guiding a cutting blade during surgical manipulation of the sclera and a system including a cutting blade and a device for guiding the cutting blade during surgical manipulation of the sclera. Also, a method for using a depth-control cutting blade and a device for guiding the cutting blade to perform surgical manipulation of the sclera in a desired shape, size, and thickness.

## Description

### RELATED APPLICATION

This application claims the priority of U.S. provisional application Ser. No. 62/745,155, filed October 12, 2018, which is incorporated herein by reference in its entirety for all purposes.

### FIELD OF THE DISCLOSURE

The field of disclosure relates to devices for use during ophthalmic surgery. More particularly, the field of disclosure relates to devices for performing surgical manipulation of the sclera during surgical procedures performed on the eye.

### BACKGROUND OF THE DISCLOSURE

Glaucoma, the leading cause of irreversible blindness in the world, is a group of diseases affecting the optic nerve, frequently characterized by increased intraocular pressure (i.e., pressure within the eye). Patients suffering from glaucoma are typically initially managed with medical therapy. However, some patients are unable to tolerate medication or do not adhere to their drug regimens, and, consequently, may require surgical intervention to preserve their vision. In some cases, when glaucoma continues to progress despite the use of medication regimes and/or laser treatments (e.g., laser trabeculoplasty), a glaucoma filtration procedure (e.g., trabeculectomy, non-penetrating deep sclerectomy, etc.) may be recommended.

Most glaucoma filtration surgical techniques (e.g., trabeculectomy, non-penetrating deep sclerectomy, etc.) involved an initial required step of performing scleral flap, in which one-third to one-half of the scleral thickness (i.e., the thickness of the sclera, which is the white outer layer of the eye) is dissected to the clear cornea. The procedure of manually performing scleral flap with manual surgical instruments (e.g., blades, knives, etc.) is both technically demanding and time-consuming to perform properly (i.e., to create a flap that is uniform and has the appropriate thickness and size). There are many risks and complications associated with the scleral flap procedure which can impact the outcome of the procedure. For example, too deep or too shallow thickness of the scleral flap can influence both the short-term and the long-term outcome of the procedure. In addition, rupture of the scleral flap during preparation of the scleral flap can prevent using the necessary control on aqueous outflow using uniform and/or complete scleral flap covering/closure on the filtration area.

Too deep of a scleral flap during glaucoma procedure such as trabeculectomy or non-penetrating deep sclerectomy is leading to generation of a thick flap that is applying excessive load and pressure on the filtration area directly or its pathway that leading to failure of the surgical procedure; the too deep flap is too thick and can provide excessive resistance to aqueous outflow due to the increased weight of the sclera in the flap on the aqueous outflow that can blocks the filtration area directly or the aqueous outflow. Additionally, too deep can result in penetration into the anterior chamber or the choroid layer, requires converting a non-penetrating surgery into a penetrating procedure (e.g., trabeculectomy). Exposure of the choroid or the ciliary body can result in excess inflammatory reaction that can affect the outcome of the procedure. For example, such inflammation can accelerate the scarring process, resulting in limiting the duration of effectiveness of the surgery.

Too thin scleral flap can prevent the provision of the necessary resistance to outflow when performing aggressive penetrating procedure (e.g. trabeculectomy), and the risk for hypotony-related complications is higher. In addition, when performing CO2 Laser Assisted Deep Sclerectomy, which is laser-based non-penetrating surgery by ablating the sclera, the key for successful operation is anatomical identification for the correct scan pattern positioning. However, when too thin a scleral flap is present, the anatomical landmarks are not clear and can result in misplacement of the scan pattern. Additionally, too thin of a scleral flap can result in non-effective sutures required for closure of the scleral flap above the filtration area. Thus, the required controlled resistance to aqueous outflow with the scleral flap cannot then be achieved.

Scleral flap of non-uniform thickness can have negative consequences for CO2 Laser Assisted Sclerectomy. In this procedure, the laser that is used ablates equally in all areas of a scanning pattern. Thus, if the scleral flap is non-uniform, the ablations will keep the same non-uniformity of the tissue, resulted in areas that are deep ablated and exposed, and in areas that are less ablated and therefore less exposed; to get the optimal result of even exposure, the non-uniform scleral flap leading to excessive required ablations, and is causing to elongation of the procedure with the laser system.

Cataracts are the leading cause of blindness worldwide with the majority of cases in developing nations. The leading techniques for performing cataract surgery are extracapsular cataract extraction (ECCE), small incision cataract surgery (SICS), and phacoemulsification. The most common technique, phacoemulsification, has become the standard of care, but requires very expensive phacoemulsification equipment, which may not be available at all centers, especially in developing countries.

Manual scleral flap during cataract surgery is required in places where the availability of phacoemulsification equipment is limited, or in cases when the phacoemulsification surgery requires conversion into a manual technique (ECCE or SICS) due to complications, or when the cataract condition (i.e. too rigid lens) is not effective for the use of phacoemulsification surgery due to limitations of phacoemulsification equipment; in such cases it is required for the surgeon to perform a manual technique (ECCE or SICS), and understand the key principles for these manual procedures.

ECCE is a technique in which a portion of the anterior capsule of the lens is removed, allowing extraction of the lens nucleus and cortex, leaving the remainder of the anterior capsule, the posterior capsule, and the zonular support intact. This is the case in all the present cataract surgery techniques (i.e., ECCE, SICS and phacoemulsification). By convention, however, ECCE refers to a procedure in which the intact lens nucleus is removed from the eye through a scleral flap including limbal incision (i.e. incision is made in the limbus line), which include the following dimensions: it is about 10-12 mm in standard extracapsular surgery, about 5.5- 7.0 mm in manual small incision surgery ("MSICS"), and about 3-5.5 mm in instrumental phacoemulsification, depending upon the technique and implant.

One of the key surgical steps in SICS and ECCE, which dramatically influences the efficacy and safety of the manual procedures, is the scleral incision or scleral tunnel (also called the sclerocorneal pocket), which relies upon intraocular pressure to close the internal lip of the wound, thereby creating a self-sealing wound and eliminating post-operative suture-induced astigmatism. Wound construction is of vital importance in SICS and ECCE. The ultimate outcome and the ease of delivering the nucleus are dependent on wound architecture.

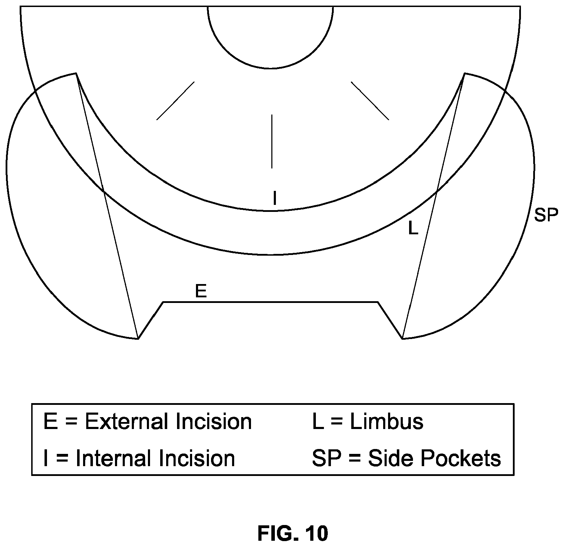

SICS in comparison to ECCE, requires a smaller scleral incision, and its scleral closure performed with no sutures (sutureless); In comparison ECCE requires much bigger scleral opening, and therefore requires sutures for its closure. Two important aspects of the incision for SICS are the self-sealing nature of the wound and the possibility of induced astigmatism. For a wound to be self-sealing, it should conform to the principle of "quadrangular or square incisional geometry." This is a general concept which states that an ideal self-sealing wound has a length equal to its width. However, the length is usually smaller, and there is a relationship between the probability that a wound is self-sealing and its approximation to quadrangular or square geometry. There are two incisions in the scleral tunnel wound for SICS: the external scleral incision and the internal corneal incision. The external wound dimensions have an important bearing on the self-sealing nature of the wound, with smaller incisions being more reliably self-sealing than larger ones. A small external wound, however, presents an obstacle to the delivery of the nucleus and IOL implantation. Hence, the incision should be fashioned in a way which lends itself to stretching. The dimension of the wound depends upon the preoperative assessment of the nucleus size and the technique used to deliver the nucleus (phacosandwich or snare or pre-chopper will help reduce incision size, whereas use of wire vectis will require a larger incision width). There are two commonly-used types of external incisions. The frown incision is a parabolic groove convex towards the limbus with the center being 1.5-2 mm behind the limbus and chord length being 6-7mm. The other incision is the straight scratch incision 5 - 6.5 mm in length, 1.5 mm behind the limbus, and with backward extensions at both ends creating side pockets. The internal incision is in the cornea and does not lend itself to stretching. Consequently, it should be made large enough to accommodate the nucleus or the implant. Generally, an 8-9 mm inner lip suffices. The internal incision should be parallel to the limbus. It should extend to the limbus but should not cut the limbus on either side. These features give the maximal security and anti-astigmatic effect to the wound. FIG. 10 shows anatomy of two types of incisions. The upper portion of FIG. 10 shows anatomy of a "frown" incision. The lower portion of FIG. 10 shows a straight incision with backward extensions that are almost perpendicular to the limbus.

Consequently, the properties of a self-sealing wound for SICS include an approximation of quadrangular or square geometry, a relatively small external incision with a geometrical shape that lends itself to stretching, and a tunnel that flares to a larger internal incision. The self-sealing is ensured by the remoteness of the two incisions brought about by the collapsed tunnel in normal conditions.

The other important aspect of wound construction is the induced astigmatism caused by the wound, which should be minimal, and if possible favorable, to counteract existing astigmatism. Corneal wounds cause the greatest, limbal intermediate, while scleral wounds cause least astigmatism. Temporal wounds are purported to cause less astigmatism than superior wounds, and have a counterbalancing effect on the natural ATR shift that occurs with age. However, most surgeons are familiar with the superior location, and the larger wound size and conjunctival dissection in SICS make this site less appropriate for wound placement. An important concept in understanding incision design in SICS is that of the incisional funnel. This is an area bounded by a pair of curvilinear lines whose shape is based upon the relationship between astigmatism and two characteristics of the incision - length and distance from limbus. Incisions made within this funnel are astigmatically stable. Short linear incisions made close to the limbus and longer incisions farther away are equally stable. A frown incision or a chevron-shaped incision incorporate a larger incision into this funnel, and hence are more desirable. Though moving farther away from the limbus makes an incision more stable, it increases the surgical difficulty by limiting access and maneuverability. Clear corneal tunnels have significant demerits: difficulty in obtaining quadrangular or square geometry due to limited length of tunnel, difficulty in anterior chamber manipulation or "oarlocking," and less security due to long healing time and lack of fibrosis.

The scleral tunnel must be constructed while a proper plane of dissection is maintained and wound architecture is not destroyed. The desired size of the external wound is measured with a caliper and marked behind the limbus. The frown convexity should be 1-1.5 mm behind the limbus and the ends about 3mm. Similarly, the ends of the straight incision lie about 2 mm behind the limbus. The external incision is about 0.3 mm deep and should be of uniform depth. Conscious effort is made to tilt the knife along the contour of the cornea to maintain uniformity of the tunnel roof and avoid buttonholing laterally. Complications can occur at each of the above steps while making the tunnel incision. Too superficial an external wound will cause a thin superficial flap or may result in buttonholing of the flap. In such a situation, the groove can be deepened and dissection started at a deeper plane without compromising the tunnel. Too deep a groove will increase the difficulty of corneal dissection and increase the chances of a premature entry into the anterior chamber. Scleral disinsertion can occur due to a deep groove and will result in large amount of postoperative ATR astigmatism. In both situations, radial sutures are necessary to ensure secure wound closure and prevent postoperative astigmatism. A tear of the lateral edge of the external wound is common, especially when too much force is being applied during dissection due to a blunt instrument. This happens due to the slight anterior rotation of the crescent knife during anterior dissection. If large, this can be repaired at the end of the surgery with 10-0 suture. Undue haste during tunnel dissection can lead to a premature entry into the anterior chamber. This leads to compromise of the features of the tunnel and creates problems during surgery like iris prolapse, etc. This is best prevented. It may become necessary to close the wound and initiate a new one at another site. If the inner lip has not been properly fashioned, it is better to use sutures to close the incision to prevent postoperative complications. A blunt keratome while making the internal incision or side port entry can cause a Descemet's membrane detachment. In such an eventuality, the membrane must be uncurled and kept in position using an air bubble tamponade. It may be sutured back in place if the detachment is large. Blunt instruments necessitating the use of undue force can lead to sudden uncontrolled entry into the AC and injury to the iris and lens, including zonular dialysis.

### BRIEF DESCRIPTION OF THE FIGURES

Some embodiments of the disclosure is herein described, by way of example only, with reference to the accompanying drawings. With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.
- FIG. 1A: shows an exemplary device with a vacuum attachment mechanism;
- FIG. 1B: shows an exemplary device with a plurality of anchoring elements
- FIG. 1C: shows an exemplary device that does not require a vacuum attachment mechanism.
- FIG. 2A: shows a top perspective view of a guidance element of an exemplary device;
- FIG. 2B: shows a bottom perspective view of the guidance element of FIG. 2A;
- FIG. 2C: shows cooperation of a guidance element and a blade to control depth of a horizontal cut;
- FIG. 2D: shows cooperation of a guidance element and a blade to control depth of a vertical cut;
- FIG. 3: shows a top perspective view of a guidance element of an exemplary device
- FIG. 4: shows a surgical ophthalmic blade that may be used in connection with the exemplary device;
- FIG. 5A: shows a top perspective view of the exemplary device of FIG. 1 receiving the surgical ophthalmic blade of FIG. 4 at a first stage of an exemplary method;
- FIG. 5B: shows a bottom perspective view of the exemplary device and surgical ophthalmic blade of FIG. 5A at the first stage of the exemplary method;
- FIG. 5C: shows a bottom perspective view of the exemplary device and surgical ophthalmic blade of FIG. 5A at a second stage of the exemplary method;
- FIG. 5D: shows a top perspective view of the exemplary device and surgical ophthalmic blade of FIG. 5A at the second stage of the exemplary method;
- FIG. 6: shows an exemplary embodiment of an auto-retractable syringe that may be coupled to an exemplary device;
- FIG. 7: shows various views of a guidance element of an exemplary device;
- FIG. 8: shows a guidance element of an exemplary device, the guidance element including an arc-shaped portion configured to overlay a limbus line to align the device;
- FIG. 9: shows a guidance element of an exemplary device, the guidance element being configured to be attached to a handle portion at a side of the guidance element;
- FIG. 10: shows anatomy of two types of incisions that are commonly used in cataract surgery;
- FIG. 11: shows a top view of a portion of an embodiment of a guidance device;
- FIG. 12: shows a bottom view of a portion of the guidance device of FIG. 11;
- FIG. 13: shows a side view of a portion of the guidance device of FIG. 11;
- FIG. 14: shows a top view of the guidance element of the guidance device of FIGs. 11-13;
- FIG. 15: shows a side view of the guidance element of FIG. 14;
- FIG. 16: shows a front view of the guidance element of FIG. 14;
- FIG. 17: shows a front cross-sectional view of the guidance element of FIG. 14;
- FIG. 18: shows a side cross-sectional view of the guidance element of FIG. 14;
- FIG. 19: shows a first top perspective view of the guidance element of FIG. 14;
- FIG. 20: shows a second top perspective view of the guidance element of FIG. 14;
- FIG. 21: shows a bottom perspective view of the guidance element of FIG. 14;
- FIG. 22: is shows a surgical knife that may be used in connection with the guidance device of FIGs. 11-21;
- FIG. 23: shows a first end of the surgical knife of FIG. 22;
- FIG. 24: shows a second end of the surgical knife of FIG. 22;
- FIG. 25: shows the first end of the surgical knife of FIGs. 22-24 and the guidance device of FIGs. 11-21 positioned in relation to one another to make a horizontal cut;
- FIG. 26: shows the surgical knife and the guidance device of FIG. 25 positioned with respect to the eye;
- FIG. 27: shows a close-up view of the surgical knife and the guidance device of FIG. 25 positioned with respect to the eye;
- FIG. 28: shows the second end of the surgical knife of FIGs. 22-24 and the guidance device of FIGs. 11-21 positioned in relation to one another to make a cut between layers of the sclera;
- FIG. 29: shows an alternative perspective of the surgical knife and the guidance device of FIG. 28;
- FIG. 30: shows the surgical knife and the guidance device of FIG. 28 positioned with respect to the eye;
- FIG. 31: shows a close-up view of the surgical knife and the guidance device of FIG. 28 positioned with respect to the eye;
- FIG. 32: shows an alternative perspective close-up view of the surgical knife and the guidance device of FIG. 28 positioned with respect to the eye;
- FIG. 33: shows the first end of the surgical knife of FIGs. 22-24 and the guidance device of FIGs. 11-21 positioned in relation to one another to make a vertical cut; and
- FIG. 34: shows the surgical knife and the guidance device of FIG. 33 positioned with respect to the eye.

### SUMMARY OF THE DISCLOSURE

The exemplary embodiments relate to a device for guiding a cutting blade during surgical manipulation of the sclera. The exemplary embodiments also relate to a system including a cutting blade and a device for guiding the cutting blade during surgical manipulation of the sclera (alternately referred to herein as a "guidance device"). The exemplary embodiments also relate to a method for using a depth-control cutting blade and a device for guiding the cutting blade to perform surgical manipulation of the sclera in a desired shape, size, and thickness.

In an embodiment, a guidance device for performing surgical manipulation of a sclera of an eye of a patient includes a guidance element configured to be positioned adjacent to the sclera, the guidance element including a horizontal cutting slot configured to receive a cutting blade and guide motion of the cutting blade so as to make a horizontal cut through the sclera, the guidance element also including a blade entrance slot configured to receive the cutting blade and guide motion of the cutting blade so as to make a layering cut parallel to the sclera and through a portion of the sclera, thereby separating a desired layer of separated sclera tissue from the rest of the underlying sclera. In an embodiment, the separated sclera tissue has a quadrilateral profile. In an embodiment, the quadrilateral profile is rectangular. In some embodiments, the rectangular profile has a height in the range of 1 mm to 6 mm and a width in the range of 2 mm to 12 mm.

In an embodiment, the guidance device also includes first and second vertical cutting slots, each of the first and second vertical cutting slots configured to receive the cutting blade and guide motion of the cutting blade so as to make corresponding first and second vertical cuts through the sclera, the first and second vertical cuts being parallel to one another and perpendicular to the horizontal cut, whereby the first vertical cut, the second vertical cut, the horizontal cut, and the layering cut define a scleral flap having desired dimensions. In an embodiment, the scleral flap has a quadrilateral profile. In an embodiment, the quadrilateral profile is a rectangular profile. In an embodiment, the rectangular profile is a square profile. In an embodiment, the square profile has an edge length of between 2 millimeters and 5 millimeters. In an embodiment, the rectangular profile has a height in the range of 1 mm to 6 mm and a width in the range of 2 mm to 12 mm. In an embodiment, the quadrilateral profile is a trapezoidal profile. In an embodiment, the trapezoidal profile has a height in the range of 1 mm to 6 mm and a maximum width in the range of 2 mm to 12 mm.

In an embodiment, the guidance device includes a vacuum mechanism configured to apply a suction force to the sclera so as to pull the sclera upward for a reproducible blade penetration within the tissue, for facilitating movement of the cutting blade, and for reproducible cutting of the desired layer thickness from the rest of the underlying tissue. In some embodiments, the vacuum mechanism is integrated into the guidance device. In some embodiments, the vacuum mechanism includes a connection to an external vacuum source.

In some embodiments, the guidance device includes a handle. In some embodiments, the handle is positioned adjacent to the horizontal cutting slot. In some embodiments, the guidance device includes first and second vertical cutting slots, and the handle is positioned adjacent to the first vertical cutting slot.

In some embodiments, the guidance device includes anchoring elements configured to retain the guidance device in proximity to the sclera. In some embodiments, the anchoring elements include needles. In some embodiments, the anchoring elements include pins.

In some embodiments, the guidance device includes an aiming mechanism configured to facilitate correct positioning of the guidance device with respect to the eye. In some embodiments, the aiming mechanism includes an arc-shaped portion. In some embodiments, the arc-shaped portion is configured so as to overlay a limbus line when the guidance device is correctly positioned with respect to the eye.

Some exemplary embodiments relate to a kit comprising the guidance device and one or more surgical knives. In some embodiments, the one or more surgical knives comprise two separate knives each with a single blade. In some embodiments, the single blades are the same. In some embodiments, the single blades are different. In some embodiments, the one or more surgical knives can comprise a single double-ended surgical knife. In some embodiments, each blade of the double-ended surgical knife is the same. In some embodiments, each blade of the double-ended surgical knife is different.

### DETAILED DESCRIPTION OF THE DISCLOSURE

Among those benefits and improvements that have been disclosed, other objects and advantages of this disclosure will become apparent from the following description taken in conjunction with the accompanying figures. Detailed embodiments of the present disclosure are disclosed herein; however, it is to be understood that the disclosed embodiments are merely illustrative of the disclosure that may be embodied in various forms. In addition, each of the examples given in connection with the various embodiments of the disclosure which are intended to be illustrative, and not restrictive.

Throughout the specification and claims, the following terms take the meanings explicitly associated herein, unless the context clearly dictates otherwise. The phrases "in one embodiment," "in an embodiment," and "in some embodiments" as used herein do not necessarily refer to the same embodiment(s), though it may. Furthermore, the phrases "in another embodiment" and "in some other embodiments" as used herein do not necessarily refer to a different embodiment, although it may. Thus, as described below, various embodiments of the disclosure may be readily combined, without departing from the scope or spirit of the disclosure.

As used herein, the term "based on" is not exclusive and allows for being based on additional factors not described, unless the context clearly dictates otherwise. In addition, throughout the specification, the meaning of "a," "an," and "the" include plural references. The meaning of "in" includes "in" and "on."

In some embodiments, a system includes an ophthalmic surgical blade and a device for guidance of the surgical blade. In some embodiments, the ophthalmic surgical blade is a crescent blade. In some embodiments, the guidance device is configured to guide the surgical blade to perform a cut having the required depth and dimensions of the scleral flap. In some embodiments, the guidance device is configured to maintain the blade in an orientation substantially parallel to the surface of the eye and to prevent angular movement of the blade upward or downward, thereby ensuring the generation of a uniform flap (i.e., preventing the generation of too thick or thin of a flap). In some embodiments, the guidance device is configured to define the thickness of the scleral flap by directing the blade entrance into the tissue at specific depth/thickness. In some embodiments, the guidance device is configured to affect the shape and size of the flap. In some embodiments, the guidance device includes slots for directing knife working positions, and is thereby configured to dictate the size and dimensions of the flap. In various embodiments, slots can be differently sized and shaped so as to be configured to enable generation of the scleral flap in the desired shape (e.g., rectangle, square, trapezoidal) and dimensions.

In some embodiments, a method includes placing a guidance device on a patient's eye at a location where a scleral flap is to be created, whereby anchoring elements anchor the guidance device to the patient's eye; operating a vacuum mechanism to facilitate entry of a blade into the sclera of the patient's eye; inserting a blade into a blade entrance slot of the guidance device; moving the blade within the blade entrance slot to create a bottom of a layer of the sclera; removing the blade from the blade entrance slot; placing the blade in a horizontal slot of the guidance device; moving the blade within the horizontal slot to define an end of the scleral flap; removing the blade from the horizontal slot; placing the blade in a first vertical slot of the guidance device; moving the blade within the first vertical slot to define a first side of the scleral flap; removing the blade from the first vertical slot; placing the blade in a second vertical slot of the guidance device; moving the blade within the second vertical slot to define a second side of the scleral flap; and removing the blade from the second vertical horizontal slot; placing the blade in a first vertical slot of the guidance device; whereby the bottom of the layer, the first side, the second side, and the end define a scleral flap.

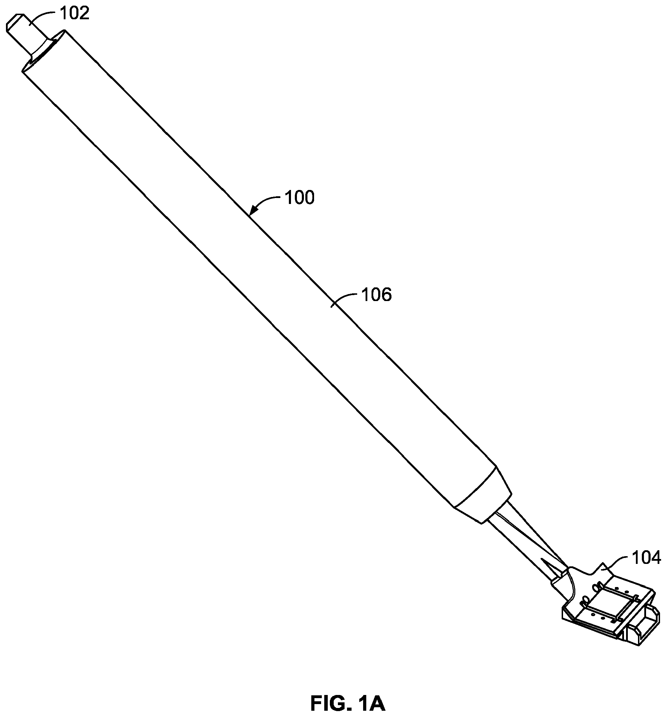

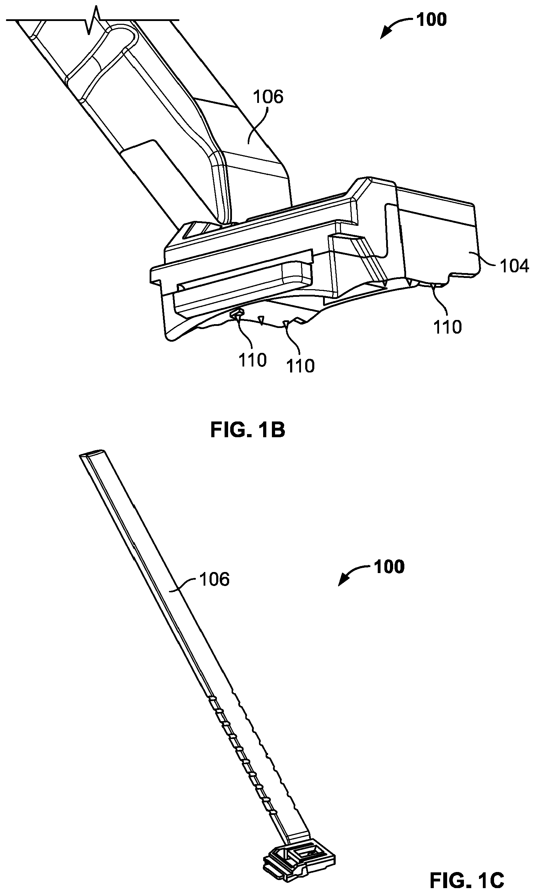

FIG. 1A shows an exemplary embodiment of a guidance device 100. As shown in FIG. 1, the guidance device 100 includes a guidance element 104 that will be described in greater detail hereinafter. In the embodiment of FIG. 1A, the guidance device 100 includes a connector 102 to an external vacuum source. In some embodiments, a guidance device includes an internal vacuum source (e.g., a vacuum pump). The vacuum source is configured to cooperate with a guidance element 104 to attach the guidance device 100 to the eye during surgery. As further shown in FIG. 1A, the guidance device 100 includes a handle 106 positioned intermediate the connector 102 and the guidance element 104

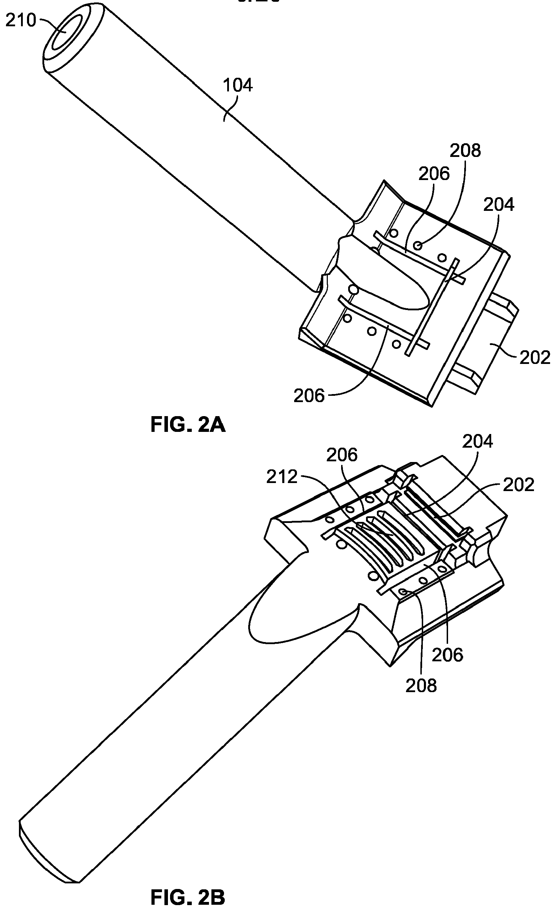

In some embodiments, the guidance element 104 is not configured to cooperate with a vacuum source to attach the guidance device 100 to the eye during surgery. Accordingly, in some embodiments, such as the embodiment of FIG. 1B the guidance element 104 can instead comprise a plurality of anchoring elements 110 (e.g., needles, pins, etc.), which are configured to attach the guidance element 104 to the eye during surgery. Accordingly, due to the presence of anchoring elements 110, in some embodiments, such as the embodiment of FIG. 1C, there is no connector to an external vacuum source on the handle 106. FIG. 2A shows a detailed view of the guidance element 104 of the guidance device 100. The guidance element 104 includes a blade entrance slot 202. In some embodiments, the blade entrance slot 202 includes a slot height that is configured to position the blade so as to make a cut parallel to the surface of the eye that will create a scleral flap of a required thickness. The average thickness of the sclera near the limbus area (0-6mm) is typically about 550 microns. In some embodiments, the blade entrance slot 202 may be positioned so as to position the blade so as to make a cut that creates a scleral flap that is about one-third to one-half the thickness of the sclera. In some embodiments, the blade entrance slot 202 may be positioned so as to position the blade so as to make a cut that creates a scleral flap that is about 200 microns in thickness. In some embodiments, the blade entrance slot 202 may be positioned so as to position the blade so as to make a cut that creates a scleral flap that is about 300 microns in thickness. In some embodiments, the blade entrance slot 202 may be positioned so as to position the blade so as to make a cut that creates a scleral flap that is about 400 microns in thickness. In some embodiments, the blade entrance slot 202 may be positioned so as to position the blade so as to make a cut that creates a scleral flap that is in the range between 200 and 300 microns in thickness. In some embodiments, the blade entrance slot 202 may be positioned so as to position the blade so as to make a cut that creates a scleral flap that is in the range between 300 and 400 microns in thickness. In some embodiments, the blade entrance slot 202 may be positioned so as to position the blade so as to make a cut that creates a scleral flap that is in the range between 200 and 400 microns in thickness. In some embodiments, the blade entrance slot 202 is configured to prevent blade movement in a vertical direction (i.e., so as to be angled upward or downward with respect to the surface of the eye) by mechanically restricting movement of the blade.

Continuing to refer to FIG. 2A, the guidance element 104 includes a horizontal slot 204 that is configured such that the blade may be placed into and slide along the horizontal slot 204 to perform a horizontal cut. In some embodiments, a horizontal cut facilitates easier insertion into the tissue when the blade is positioned within the blade entrance slot 202. In some embodiments, a horizontal cut facilitates improved tissue attraction through a vacuum mechanism, as will be described hereinafter. The guidance element 104 includes vertical slots 206 that are oriented perpendicularly to the horizontal slot 204. In some embodiments, the vertical slots 206 and the horizontal slot 204 define the desired profile (e.g., size, shape) of a scleral flap. In various embodiments, the vertical slots 206 and the horizontal slot 204 may be differently sized in order to generate different dimensions of the scleral flap, thereby accommodating the preferences of different users (e.g., ophthalmic surgeons). In some embodiments, the vertical slots 206 and the horizontal slot 204 are positioned so as to define a scleral flap having a profile that is a 5 millimeter square. In some embodiments, the vertical slots 206 and the horizontal slot 204 are positioned so as to define a scleral flap having a profile that is a 3.5 millimeter square. In some embodiments, a scleral flap having a profile that is a 3.5 millimeter square may be suitable for the performance of trabeculectomy. In some embodiments, the vertical slots 206 and the horizontal slot 204 are positioned so as to define a scleral flap having a profile that is a 4.5 millimeter square. In some embodiments, a scleral flap having a profile that is a 4.5 millimeter square may be suitable for the performance of deep sclerectomy. In some embodiments, the vertical slots 206 and the horizontal slot 204 are positioned so as to define a scleral flap having a profile that is a square having sizes in the range between 3.5 millimeters and 5 millimeters. In some embodiments, the vertical slots 206 and the horizontal slot 204 are positioned to define a profile having a rectangular shape. In some embodiments, the rectangular shape has a height in the range of 1 millimeter to 6 millimeters and a width in the range of 2 millimeters to 12 millimeters. In some embodiments, the vertical slots 206 are not perpendicular to the horizontal slot 204, and the vertical slots 206 and the horizontal slot 204 are positioned to define a profile having a trapezoidal shape. In some embodiments, the trapezoidal shape has a height in the range of 1 millimeter to 6 millimeters and a maximum width in the range of 2 millimeters to 12 millimeters.

Continuing to refer to FIG. 2A, the guidance element 104 includes holes 208 that are configured to receive anchoring elements (e.g., needles, pins, etc.) therein. In some embodiments, the holes 208 are positioned around the perimeter of a scleral flap region defined by the vertical slots 206 and the horizontal slot 204, such that anchoring elements (e.g., needles, pins, etc.) may be placed therein and extend into the surface of the eye, thereby preventing movement of the guidance device 100 with respect to the eye while operating. The guidance element 104 includes a vacuum tube 210 configured to generate suction to the tissue layer that is being cut. In some embodiments, suction stretches the scleral tissue upward, which facilitates the cutting of the tissue.

FIG. 2B shows an opposite perspective of the guidance element 104 of FIG. 2B. The blade entrance slot 202, horizontal slot 204, vertical slots 206, and holes 208 each extend through the guidance element 104. The guidance element 104 includes suction slots 212. In some embodiments, the upper side of the suction slots 212 is sealed; consequently, the suction slots 212 are not visible in FIG. 2A. In some embodiments, the suction slots 212 are in fluid communication with the vacuum tube 210. In some embodiments, the suction slots 212 are positioned slightly above the line of movement of a blade that is slid along the blade entrance slot 202. In some embodiments, the vacuum is configured to ease the separation of the desired layer of sclera from the rest of the underlying sclera tissue.

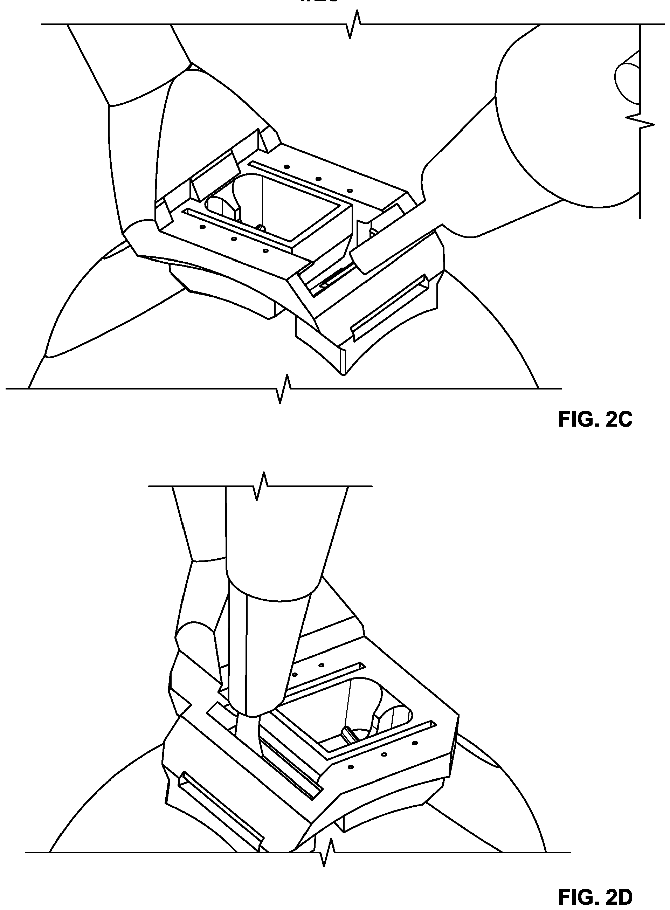

In some embodiments, the guidance element 104 is configured so as to limit the depth of cuts that are made using the horizontal slot 204 and the vertical slots 206, thereby controlling and limiting the depth of a scleral flap. In some embodiments, the horizontal slot 204 and the vertical slots 206 are configured to cooperate with a stopper that is integrated with the handle of a cutting blade so as to limit the depth of cuts. FIG. 2C shows the cooperation of the horizontal slot 204 with a stopper integrated into the handle of a cutting blade so as to limit the depth of a horizontal cut. FIG. 2D shows the cooperation of one of the vertical slots 206 with a stopper integrated into the handle of a cutting blade so as to limit the depth of a vertical cut. In some embodiments, at least one of the horizontal slot 204 or one or both of the vertical slots 206 is not configured to cooperate with a vertical stopper. Instead, in such embodiments, the entrance of a cutting blade into at least one of the horizontal slot 204 or one or both of the vertical slots 206 is configured to be blocked by a handle of the cutting blade. This blocking can occur due to the fact that the handle of the cutting blade has a thickness exceeding the thickness of the horizontal slot 204 and the vertical slots 206.

Accordingly, some embodiments of the present disclosure are configured to control the depth of the blade penetration into the guide slot, and thereby control the depth of the incision. In some embodiments, depth control is achieved through a specific mechanism, including a specific design for the length of the blade, a specific design for the slot height through which the blade is moving, the relative slot height as compared to the blade length, and control on the blade movement. For example, the handle itself or integrated stopper can be configured to enable the blade to penetrate to a certain depth in the slot and not further. By controlling the depth of the blade penetration, the depth of the incision to the target tissue can be configured to a wide range, for example, from 20 microns to 700 microns. In some embodiments this mechanism enabling blade protrusion or blade penetration or depth of incision is from 50 microns to 600 microns. in some embodiments this mechanism enabling blade protrusion or blade penetration or depth of incision is 100 microns to 500 microns. In some embodiments this mechanism enabling blade protrusion or blade penetration or depth of incision is 150 microns to 450 microns. In some embodiments this mechanism enabling blade protrusion or blade penetration or depth of incision is 200 microns to 400 microns. In some embodiments this mechanism enabling blade protrusion or blade penetration or depth of incision is 250 microns to 350 microns. In some embodiments this mechanism enabling blade protrusion or blade penetration or depth of incision is 300 microns to 325 microns. In some embodiments, the depth of incision is a fixed value falling within or corresponding to the end points of any of the aforementioned ranges. As used herein, "fixed" means that the depth cannot be adjusted by a user. In some embodiments, the depth of incision is adjustable by a user to any value falling within or corresponding to the end points of any of the aforementioned ranges.

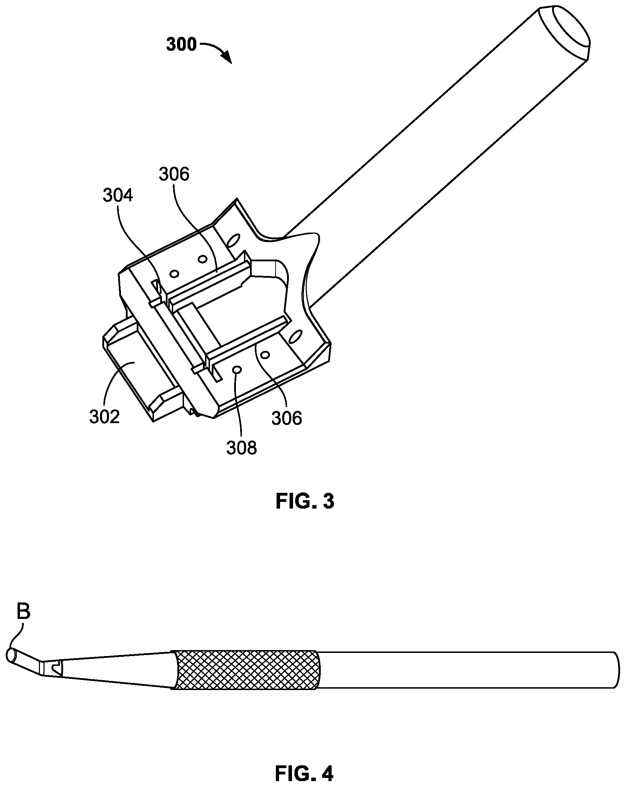

FIG. 3 shows an exemplary embodiment of a guidance device 300. The guidance device 300 of FIG. 3 lacks a vacuum arrangement, but is otherwise substantially similar to the guidance device 100 of FIGs. 1-2B. The guidance device 300 includes a blade entrance slot 302. In some embodiments, the blade entrance slot 302 includes a slot height that is configured to position the blade in so as to make a cut parallel to the surface of the eye that will create a scleral flap of a required thickness (e.g., as described above with reference to the blade entrance slot 202). In some embodiments, the blade entrance slot 302 is configured to prevent blade movement in a vertical direction (i.e., so as to be angled upward or downward with respect to the surface of the eye) by mechanically restricting movement of the blade. The guidance device 300 includes a horizontal slot 304 that is configured such that the blade may be placed into and slide along the horizontal slot 304 to perform a horizontal cut. In some embodiments, a horizontal cut facilitates easier insertion into the tissue when the blade is positioned within the blade entrance slot 302. The guidance device 300 includes vertical slots 306 that are oriented perpendicularly to the horizontal slot 304. In some embodiments, the vertical slots 306 and the horizontal slot 304 define the desired profile (e.g., size, shape) of a scleral flap. In various embodiments, the vertical slots 306 and the horizontal slot 304 may be differently sized in order to generate different dimensions of the scleral flap, thereby accommodating the preferences of different users (e.g., ophthalmic surgeons). The guidance device 300 includes holes 308 that are configured to receive anchoring elements (e.g., needles, pins, etc.) therein. In some embodiments, the holes 308 are positioned around the perimeter of a scleral flap region defined by the vertical slots 306 and the horizontal slot 304, such that anchoring elements (e.g., needles, pins, etc.) may be placed therein and extend into the surface of the eye, thereby preventing movement of the guidance device 300 with respect to the eye while operating. The blade entrance slot 302, horizontal slot 304, vertical slots 306, and holes 308 each extend through the guidance device 300.

FIG. 4 shows a surgical ophthalmic blade B. The blade B is a crescent blade with sharp edges all over its curvature and sides, which enable easy penetration into the tissue and cutting movement within the tissue. In some embodiments, the blade B includes a non-motorized handle and is fully controlled by the operator. In some embodiments, the blade B includes a motorized handle generating blade movement along the horizontal axis (i.e., parallel to the surface of the eye) for ease of cutting and for maximizing the automatization of the cutting process. In some embodiments, a system for performing scleral flap includes the guidance device 100 and the blade B.

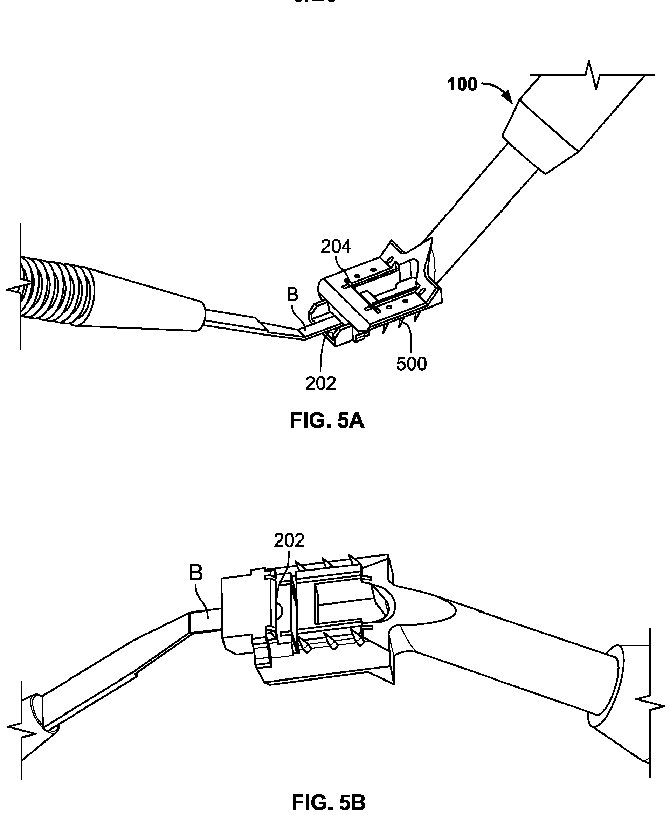

Referring now to FIGs. 5A-5D, in some embodiments, a method for performing scleral flap is performed as follows. In FIGs. 5A-5D, the suction slots 212 are omitted from the representation of the guidance device 100 for clarity of illustration. In accordance with an exemplary method, the guidance device 100 is provided with anchoring elements 500 (e.g., needles, pins, etc.). In some embodiments, the length of the anchoring elements that protrudes from the guidance device cannot exceed the depth of the sclera tissue. In some embodiments, the length of the anchoring elements cannot exceed 100 microns more than the depth of the scleral flap to be generated. In some embodiments, the guidance device 100 is configured to guide a blade to generate a scleral flap having a depth that is in a range of between 200 and 400 microns, and, accordingly, the tips of the anchoring elements 500 may protrude past the blade entrance slot 102 (i.e., the position of which defines the depth of the scleral flap) by a maximum of 100 microns. In some embodiments, the guidance device 100 with anchoring elements 500 is placed on the patient's eye in the desired location for generating a scleral flap. The anchoring elements 500 enable stable positioning of the guidance device 100 inside the sclera tissue.

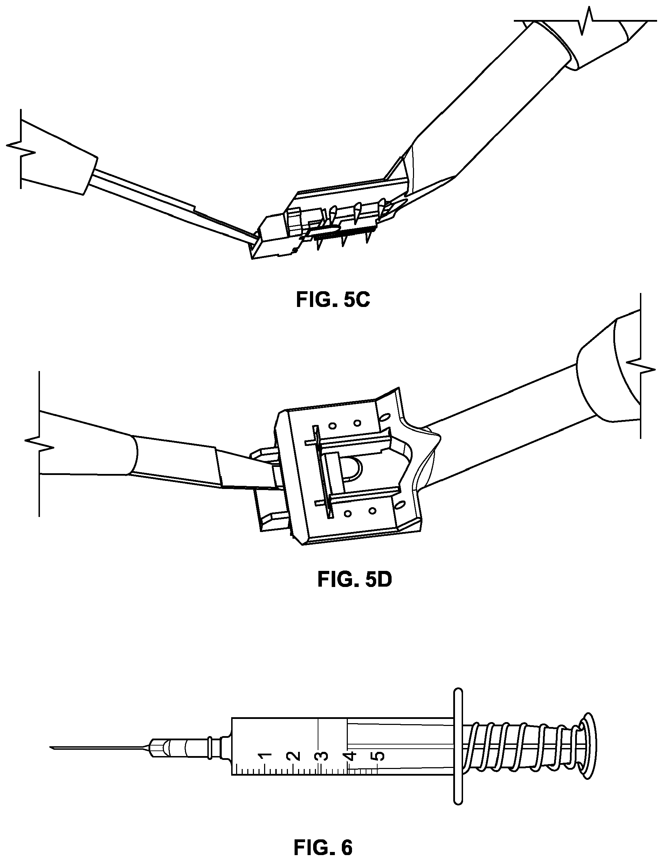

In some embodiments, the blade B is placed within the horizontal slot 204 and moved across the horizontal slot 204 to make a horizontal cut in the sclera. In some embodiments, such a horizontal cut facilitates penetration of the blade B into the sclera when the blade B is placed within the blade entrance slot 202. In some embodiments, the blade B is placed into the blade entrance slot 202, as shown in FIGs. 5A and 5B. In some embodiments, and is moved to the sides and forward and backward, as shown in FIGs. 5C and 5D, to the extent allowed by the blade entrance slot 202 to generate a tissue layer that will comprise the scleral flap. The blade entrance slot 202 maintains the blade B at a constant depth and orientation with respect to the surface of the sclera, and, therefore, ensures that the tissue layer has a uniform depth. In some embodiments, the depth is 200 microns. In some embodiments, the depth is 300 microns. In some embodiments, the depth is 400 microns. In some embodiments, the depth is in a range of between 200 microns and 300 microns. In some embodiments, the depth is in a range of between 300 microns and 400 microns. In some embodiments, the depth is in a range of between 200 microns and 400 microns.

In some embodiments, the method continues by removing the blade B from the blade entrance slot 202, inserting the blade B into one of the vertical slots 206, sliding the blade B along the one of the vertical slots 206 to make a vertical cut (i.e., along one side of the scleral flap to be generated), and then repeating this process in the other one of the vertical slots 206 (i.e., along the other side of the scleral flap to be generated). At this point, the scleral flap has been formed, and the guidance device 100 can be removed from its place on the patient's eye. In other embodiments, while the horizontal cut through the horizontal slot 204 must be made first, the remaining cutting steps (i.e., the step of forming a tissue layer by moving the blade B within the blade entrance slot 202 and the step of making vertical cuts by moving the blade B within the vertical slots 206) may be reversed in sequence.

As discussed above, in some embodiments, a guidance device (e.g., the guidance device 100) includes a vacuum mechanism. In some embodiments, the vacuum mechanism includes the connector 102, the vacuum tube 210, and the suction slots 212. In some embodiments, the vacuum mechanism is activated prior to anchoring the guidance device 100 to the patient's eye. In some embodiments, vacuum mechanism anchors the guidance device 100 to the tissue of the patient's eye. In some embodiments, the vacuum mechanism eases the blade penetration of the blade B into the tissue of the patient's eye. In some embodiments, the vacuum mechanism (e.g., by the proximity of the suction slots 212 to the patient's eye) pulls the sclera upward due to the flexibility of the tissue of the sclera. In some embodiments, when the vacuum mechanism operates in this manner, the blade B can be inserted into the blade entrance slot 202 and into the sclera without the need to perform a horizontal cut (e.g., through the use of the horizontal slot 204 in the manner described above) first. In some embodiments, the vacuum mechanism attracts and stabilizes the portion of the sclera that is to form the scleral flap, thereby easing the cutting process and improving the uniformity of the resulting scleral flap.

In embodiments including a vacuum mechanism, the vacuum mechanism can include various types of mechanisms. In some embodiments, the vacuum mechanism includes a vacuum pump integrated into the handle 106 of the guidance device 100. In some embodiments, the vacuum mechanism includes a hollow tube within the handle 106 of the guidance device and between the connector 102 and the suction slots 212, and the connector 102 is configured to be coupled to an external vacuum pump. In some embodiments, the vacuum mechanism includes an auto-retractable syringe with a spring. FIG. 6 illustrates an exemplary auto-retractable syringe with a spring. In some embodiments, an auto-retractable syringe with a spring is integrated into the handle 106 of the device 100. In some embodiments, an auto-retractable syringe with a spring is configured to apply a vacuum (e.g., to the suction slots 212) upon release of the syringe's tube/spring. In some embodiments, an auto-retractable syringe with a spring is controlled by one or more buttons integrated into the handle 106 of the device 100. In some embodiments, an auto-retractable syringe provides for repeated generation of a vacuum, if necessary, by repeatedly contracting and releasing the syringe.

In some embodiments, hollow tubes extend through the handle 106 and head 104 of the guidance device 100 in order to convey the vacuum pressure to the desired location at the surface of the patient's eye. In some embodiments, the vacuum pressure to be applied at the source of the vacuum depends on the location of the source of the vacuum relative to the target area (i.e., the surface of the patient's eye). In some embodiments, the vacuum pressure generated by the vacuum mechanism is in a range between 100 and 700 mm Hg. In some embodiments, the vacuum mechanism is configurable by configuring a flow rate of a vacuum pump. In some embodiments in which a vacuum pump is integrated with the handle 106 of the guidance device 100, the flow rate of the vacuum pump is in the range between 0.3 liters per minute and 2.0 liters per minute. In some embodiments in which a vacuum pump is an external pump that is connected to the guidance device 100 via the connector 102, the flow rate of the vacuum pump is in the range between 30 liters per minute and 40 liters per minute. In some embodiments, the flow rate of the vacuum pump is between 0.3 liters per minute and 40 liters per minute.

As noted above, the exemplary guidance device 300 is substantially similar to the exemplary guidance device 100 other than insofar as the exemplary guidance device 300 lacks a vacuum arrangement. Accordingly, it will be apparent to those of skill in the art that a method for performing scleral flap practiced with the use of the exemplary guidance device 300 will be substantially similar to the method described above with reference to the exemplary guidance 100, save for the omission of the operation of the vacuum.

In some embodiments of the disclosure, the blade B is connected to a motor configured to move the blade B along a horizontal axis (i.e., parallel to the surface intended for cutting) to facilitate an easy cutting action. In some embodiments, a motorized handle enables the connection of the crescent blade unit generated for the purpose of this step in glaucoma operation. In some embodiments, a motorized blade B enables movement along the horizontal axis to a distance of between 50 microns and 200 microns to either side. In some embodiments, restriction of the degree of movement prevents excessive movement by a user (e.g., a surgeon), which can lead to complications. In some embodiments, all devices used in the methods described above are single-use. In some embodiments, a motorized handle is reusable and a blade is single-use.

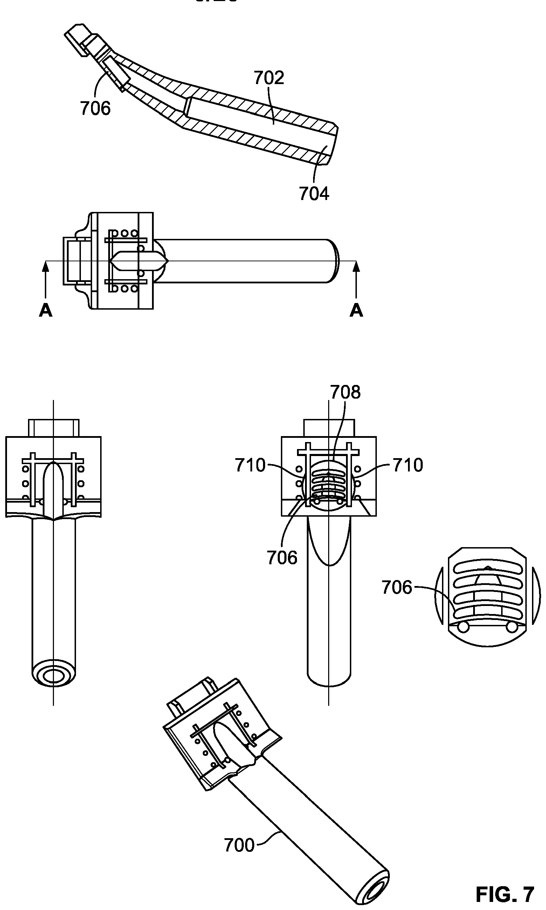

In some embodiments, the shape and size of the generated scleral flap will be controlled by the design of the vertical and horizontal slots (e.g., the horizontal slot 204 and the vertical slots 206 of the guidance device 100), which define the frame of the generated flap. FIG. 7 shows a drawing of an exemplary guidance element 700 of a guidance device 100 that includes a vacuum mechanism. The drawing of FIG. 7 includes dimensions, but it will be apparent to those of skill in the art that these dimensions are only exemplary. As shown in FIG. 7, the guidance element 700 includes a hollow internal tube 702 providing a pathway whereby a vacuum may be conveyed between, at a first end, a connector 704 connected to an external vacuum pump mechanism (not shown), and, at the other end, suction slots 706. The suction slots 706, which define an area in which a vacuum pressure is conveyed to the surface of the patient's eye, are surrounded by a horizontal slot 708 and vertical slots 710, which are substantially similar to the horizontal slot 204 and vertical slots 206 of the guidance element 104 described above. The guidance element 700 includes holes 712 configured to receive anchoring elements as described above.

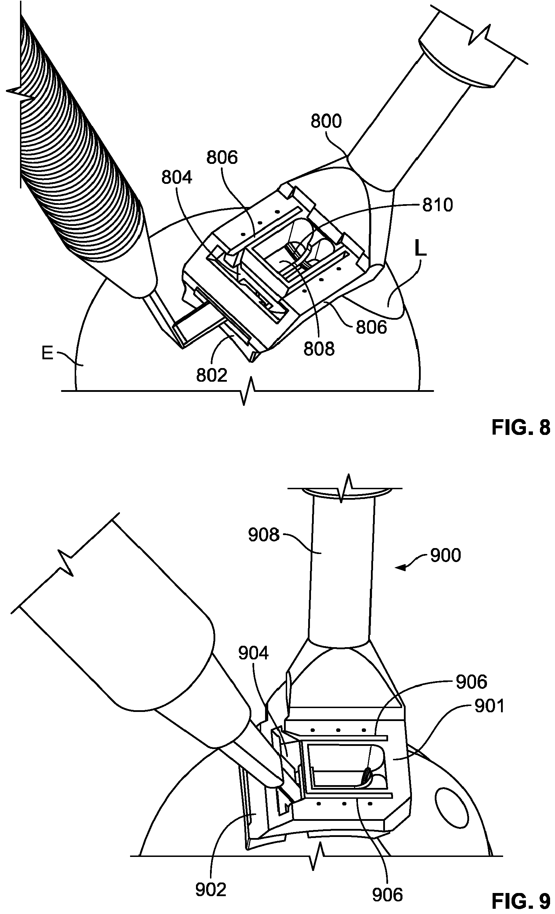

In some embodiments, a guidance device includes an arc-shaped portion that is sized, shaped and positioned so as to assist a user in properly positioning the guidance device with respect to the eye. In some embodiments, the arc-shaped portion is sized, shaped and positioned so as to overlay the limbus line (i.e., the boundary between the cornea and the sclera) when the guidance device is properly positioned with respect to the eye. FIG. 8 shows a guidance device 800 in use. In some embodiments, the guidance device 800 is substantially similar to the guidance device 300 described above other than as described hereinafter. In some embodiments, the guidance device 800 includes a blade entrance slot 802, a horizontal slot 804, and two vertical slots 806. In some embodiments, the guidance device 800 includes a window 808 and an arc-shaped portion 810 extending across the window 808. In some embodiments, the guidance device 800 is configured such that, when positioned adjacent to the patient's eye E, the eye E can be seen through the window 808 and the arc-shaped portion 810 is aligned with the limbus line L when the guidance device 800 is positioned and oriented properly with respect to the eye E. It should be noted that while the guidance device 800 lacks a vacuum apparatus, as does the guidance device 300, it will be apparent to those of skill in the art that a guidance device including a vacuum apparatus (e.g., the guidance device 100) could also be modified to include an arc-shaped portion such as the arc-shaped portion 810 of the guidance device 800.

In some embodiments, a guidance device includes a guidance element that is configured to be coupled to a handle portion at a rear of the guidance element.

For example, FIGs. 1-2B show a guidance device 100 including a guidance element 104 that is configured to be coupled to a handle portion 106 at a rear of the guidance element 104 (i.e., to a side of the guidance element 104 opposite the horizontal slot 204. As an alternative, in some embodiments, a guidance device includes a guidance element that is configured to be coupled to a handle portion at a side of the guidance element. FIG. 9 shows a guidance device 900 that is substantially similar to the guidance device 800 described above other than insofar as will be described hereinafter. In some embodiments, the guidance device 900 has a guidance element 901 including a blade entrance slot 902, a horizontal slot 904, and two vertical slots 906. In some embodiments, the guidance device 900 includes a handle connector portion 908 that is positioned to a side of the guidance element 901 (i.e., is positioned most closely to one of the vertical slots 906).

In some embodiments, a guidance device includes a horizontal slot configured for defining an initial incision within the eye (e.g., the horizontal slot 204 described above) and a blade entrance slot for separating a layer of the sclera having a desired thickness from the rest of the underlying sclera tissue (e.g., the blade entrance slot 202 described above), but lacks vertical slots. In some embodiments, such an incision may be referred to as a "groove" rather than as a "flap". In some embodiments, such a cutting device may be suitable for use in cataract surgery, such as extracapsular cataract extraction ("ECCE") or manual small incision cataract surgery ("MSICS"). In some such surgical procedures, the surgeon makes an incision in the sclera near the outer edge of the cornea (i.e., the limbus line). In some embodiments, the size of such an incision depends on whether the lens of the nucleus is to be removed whole or is to be broken into smaller pieces and removed by suction. In some such surgical procedures, the surgeon then enters the eye through the incision and opens the front of the capsule that holds the lens in place. However, in some surgical procedures such as ECCE and MSICS, no flap of the sclera is lifted, so no vertical cuts are made to define a liftable flap.

In some embodiments of a guidance device configured for use in surgical procedures such as ECCE and MSICS, the width of a groove (e.g., the length of a cut defined by a horizontal slot) is in a range between 5 mm and 11 mm. In some embodiments, the width of the groove depends on the specific surgical technique to be practiced (e.g., ECCE, MSICS, etc.). In some embodiments, the width of the groove depends on the preference of the surgeon. In some embodiments, a guidance device may be configured to facilitate the formation of a groove of a specific width (e.g., 5 mm, 6 mm, 7 mm, 8 mm, 9 mm, 10 mm, 11 mm, etc.), and guidance devices may be made in a variety of configurations to provide a variety of options of widths.

In some embodiments of a guidance device configured for use in surgical procedures such as ECCE and MSICS, the depth of a groove (e.g, the location of a cut as defined by a blade entrance slot) is in a range of between 1/3 of the depth of the sclera and 1/2 of the depth of the sclera (e.g., in a range of 200 microns to 400 microns). In some embodiments, the depth of the groove depends on the specific surgical technique to be practiced (e.g., ECCE, MSICS, etc.). In some embodiments, the depth of the groove depends on the preference of the surgeon. In some embodiments, a guidance device may be configured to facilitate the formation of a groove of a specific depth (e.g., 1/3 of the depth of the sclera, 1/2 of the depth of the sclera, etc.), and guidance devices may be made in a variety of configurations to provide a variety of options of depths.

In some embodiments of a guidance device configured for use in surgical procedures such as ECCE and MSICS, the location of a groove (e.g., the location of a cut as defined by a horizontal slot) is in a range of 0 mm to 3 mm posterior of the limbus (wherein 0 mm posterior of the limbus means a cut positioned at the limbus). In some embodiments, the location of the groove depends on the specific surgical technique to be practiced (e.g., ECCE, MSICS, etc.). In some embodiments, the location of the groove depends on the preference of the surgeon. In some embodiments, a guidance device may be configured to facilitate the formation of a groove at a specific location (e.g., at the limbus, 1 mm posterior of the limbus, 2 mm posterior of the limbus, 3 mm posterior of the limbus, etc.), and guidance devices may be made in a variety of configurations to provide a variety of options of locations of cuts.

In some embodiments of a guidance device configured for use in surgical procedures such as ECCE and MSICS, the guidance device is configured so as to guide the formation of a groove into the anterior chamber of the eye. In some embodiments, the guidance device is configured so as to guide the formation of a groove that is wider in the cornea than at the scleral opening, by using a long length blade entering through the slot 902 with the guidance element being configured not to limit movement of the blade in the anterior direction.

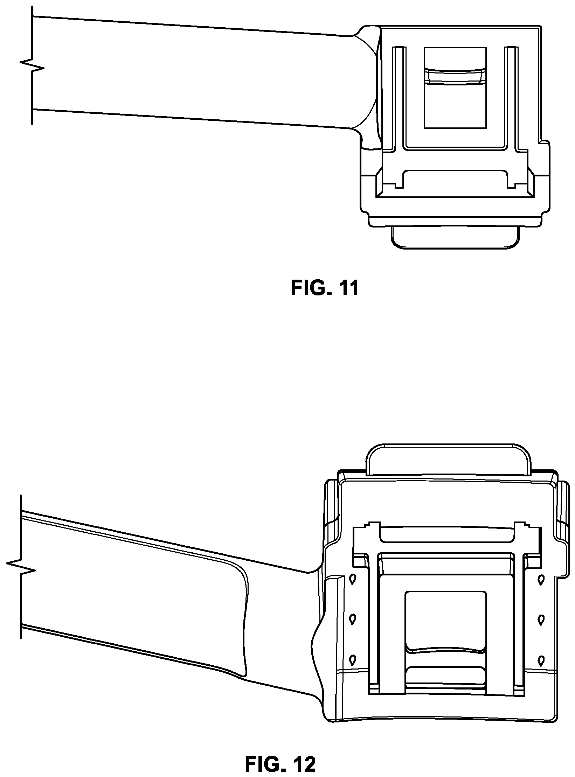

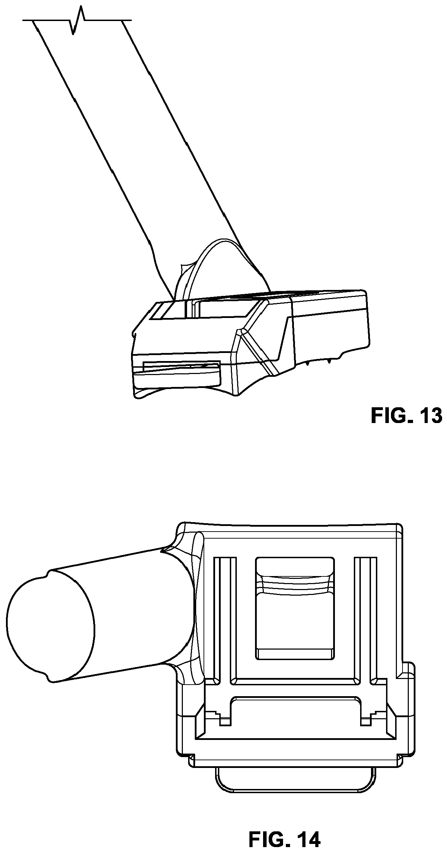

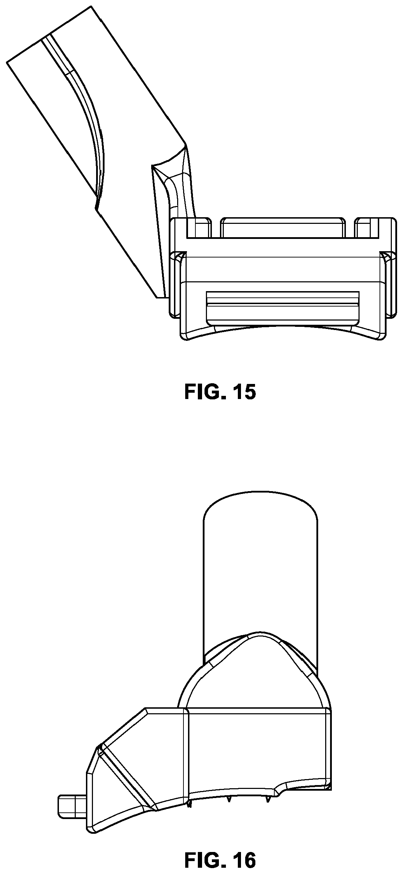

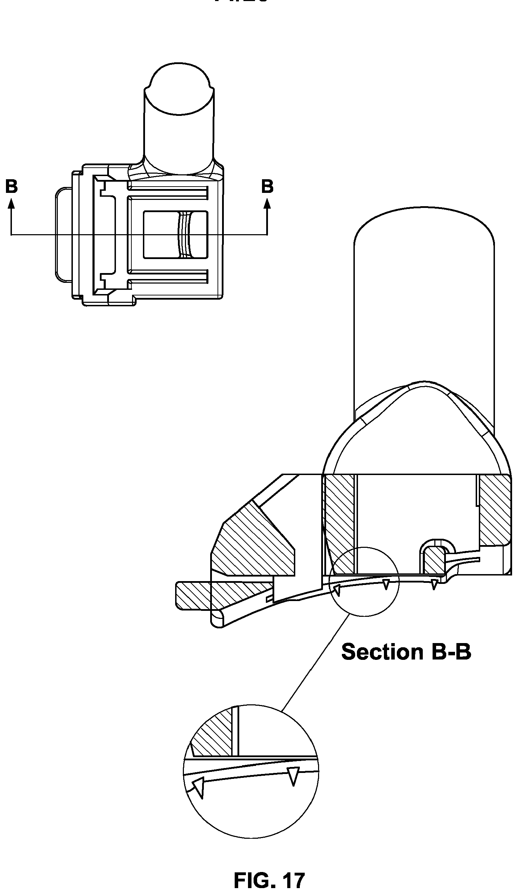

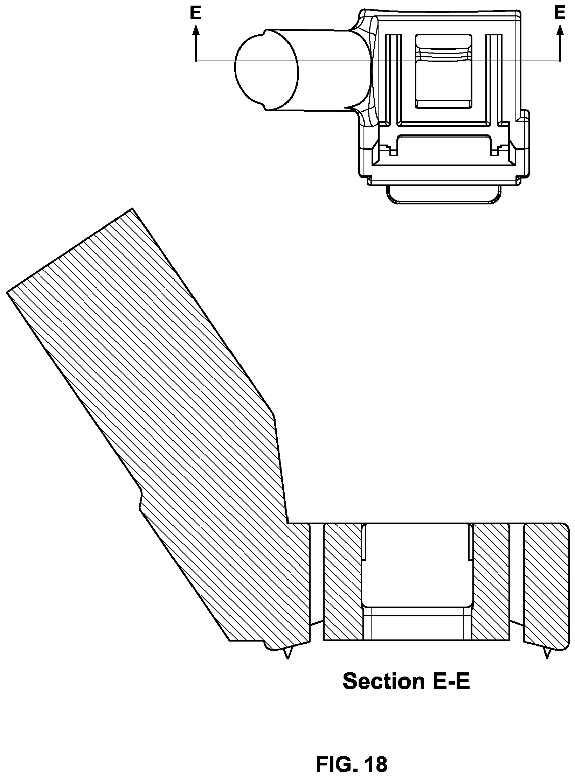

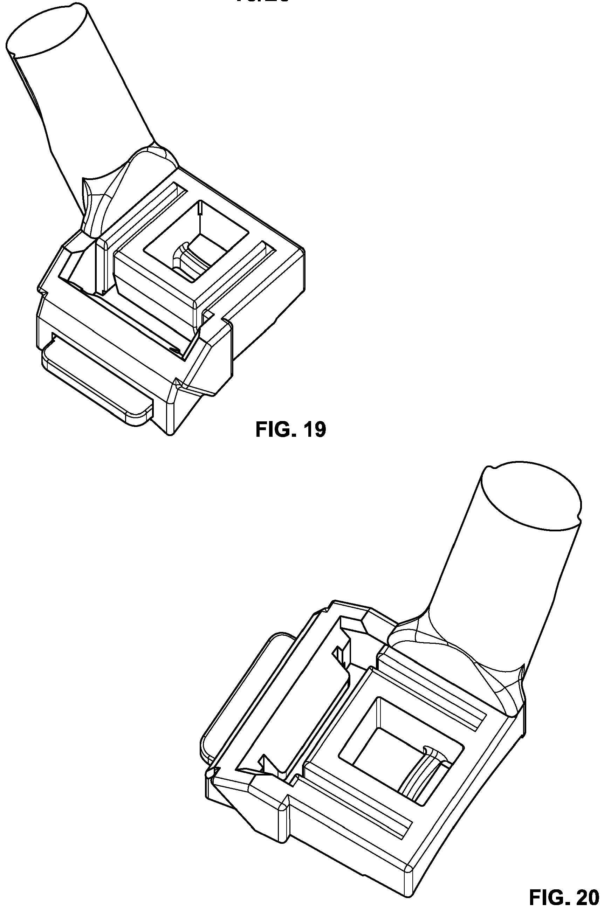

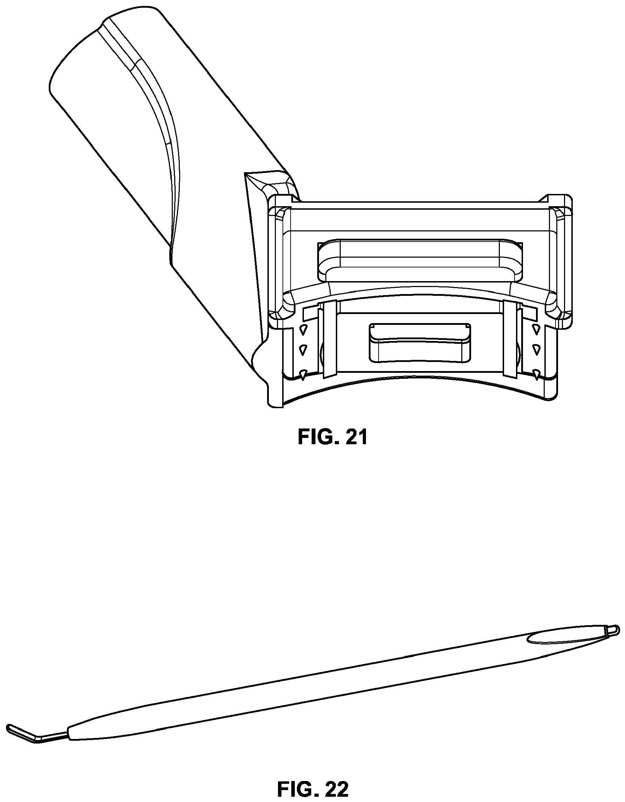

FIGs. 11-13 are illustrations of an embodiment of a guidance device. FIG. 11 shows is a top view of a portion of an embodiment of a guidance device. FIG. 12 is a bottom view of a portion of the guidance device of FIG. 11. FIG. 13 is a side view of a portion of the guidance device of FIG. 11. FIGs. 14-21 are drawings of the guidance element of the guidance device of FIGs. 11-13. The drawings of FIGs. 14-21 include dimensions, but it will be apparent to those of skill in the art that these dimensions are only exemplary. FIG. 14 is a top view of the guidance element of the guidance device of FIGs. 11-13. FIG. 15 is a side view of the guidance element of FIG. 14. FIG. 16 is a front view of the guidance element of FIG. 14. FIG. 17 is a front cross-sectional view of the guidance element of FIG. 14. FIG. 18 is a side cross-sectional view of the guidance element of FIG. 14. FIG. 19 is a first top perspective view of the guidance element of FIG. 14. FIG. 20 is a second top perspective view of the guidance element of FIG. 14. FIG. 21 is a bottom perspective view of the guidance element of FIG. 14.

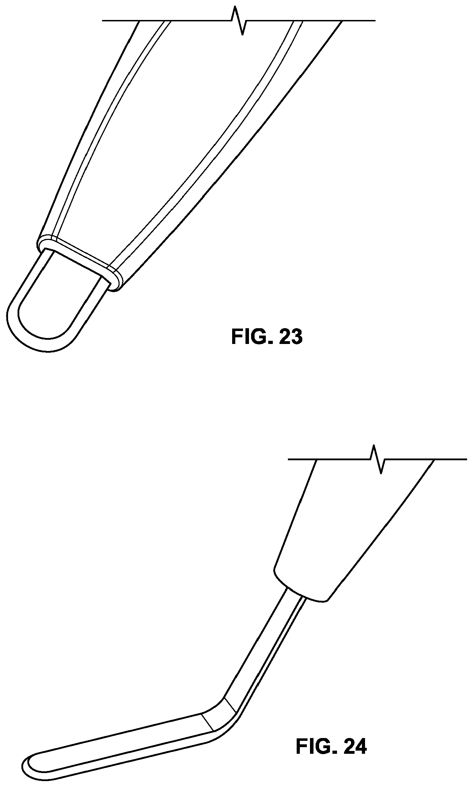

In some embodiments, the present disclosure relates to a kit comprising the guidance device and one or more surgical knives. In some embodiments, the one or more surgical knives comprise two separate knives each with a single blade. In some embodiments, the single blades are the same. In some embodiments, the single blades are different. In some embodiments, the one or more surgical knives can comprise a single double-ended surgical knife. For example, FIG. 22 is an illustration of a double-ended surgical knife that may be used as a kit in connection with the guidance device of FIGs. 11-21. In addition, FIG. 23 is a detailed view of a first end of the surgical knife of FIG. 22 that may be used as a kit in connection with the guidance device of FIGs. 11-21 to make horizontal and vertical cuts. FIG. 24 is an illustration of a second end of the surgical knife of FIG. 22 that may be used as a kit in connection with the guidance device of FIGs. 11-21 to separate layers of the sclera.

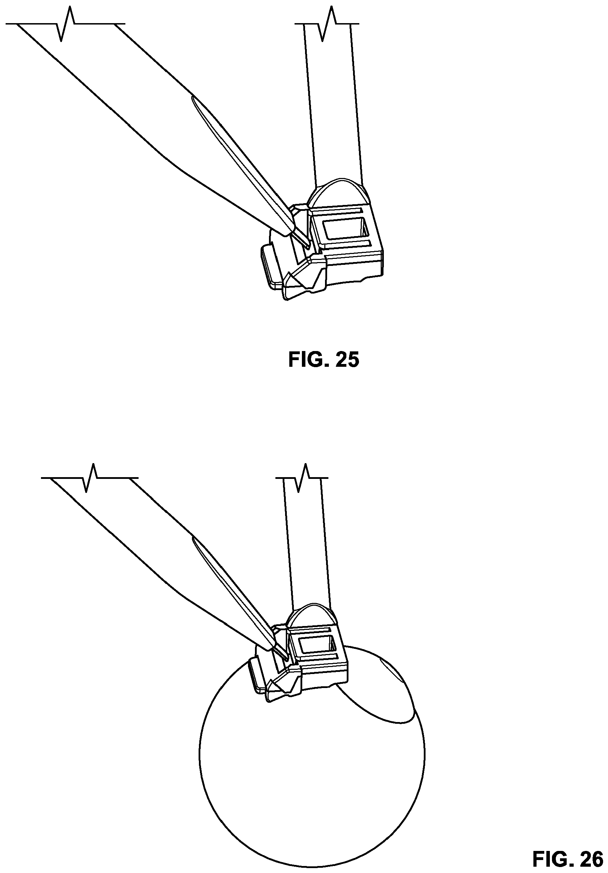

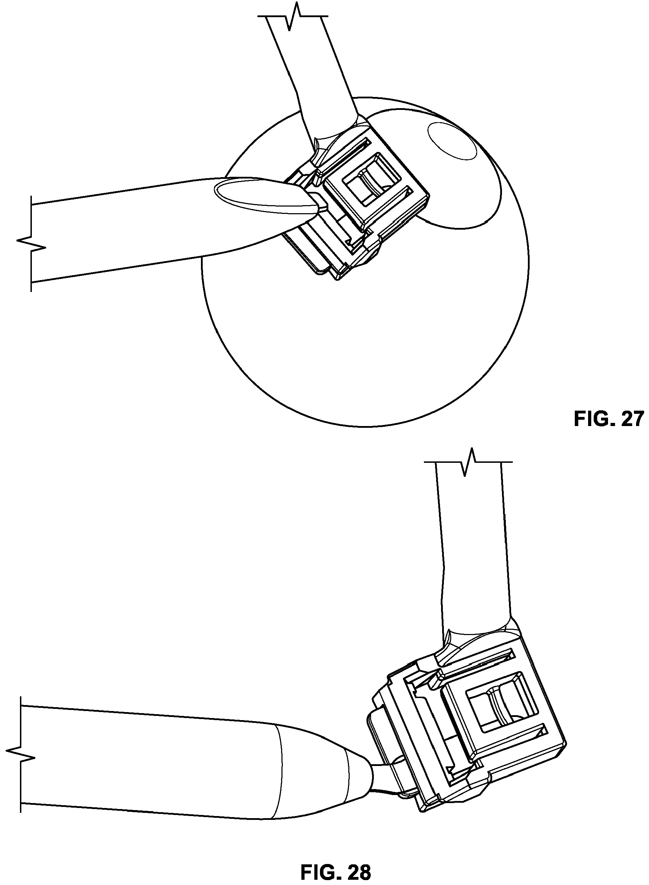

FIGs. 25-27 are illustrations of the use of the kit described herein (comprising a guidance device and a surgical knife of FIG. 22) to make a horizontal cut. FIG. 25 shows the kit with the surgical knife and the guidance device positioned in relation to one another. FIG. 26 shows the kit with surgical knife and the guidance device positioned in relation to one another and positioned with respect to the eye. FIG. 27 shows a close-up view of the kit with the surgical knife and the guidance device positioned in relation to one another and positioned with respect to the eye.

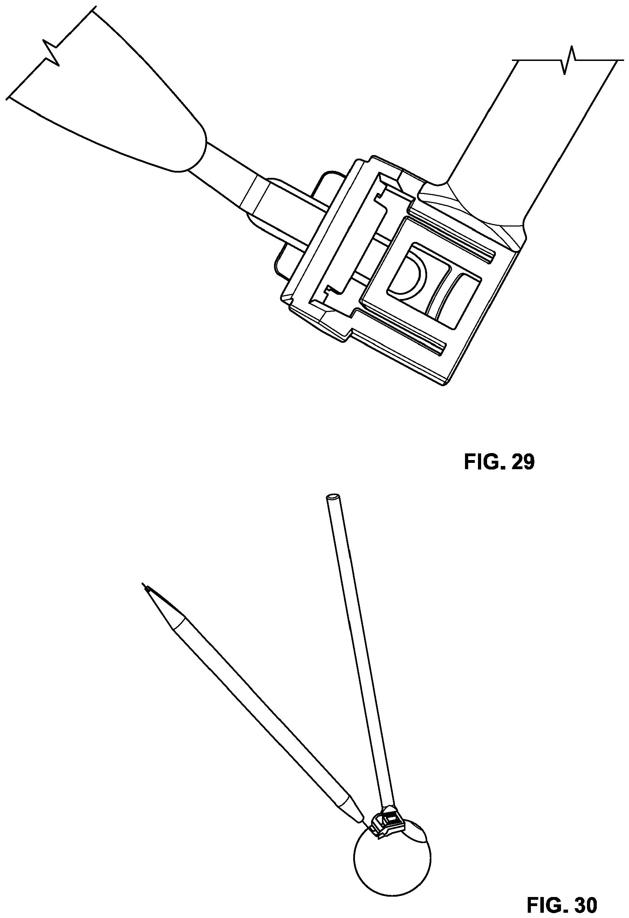

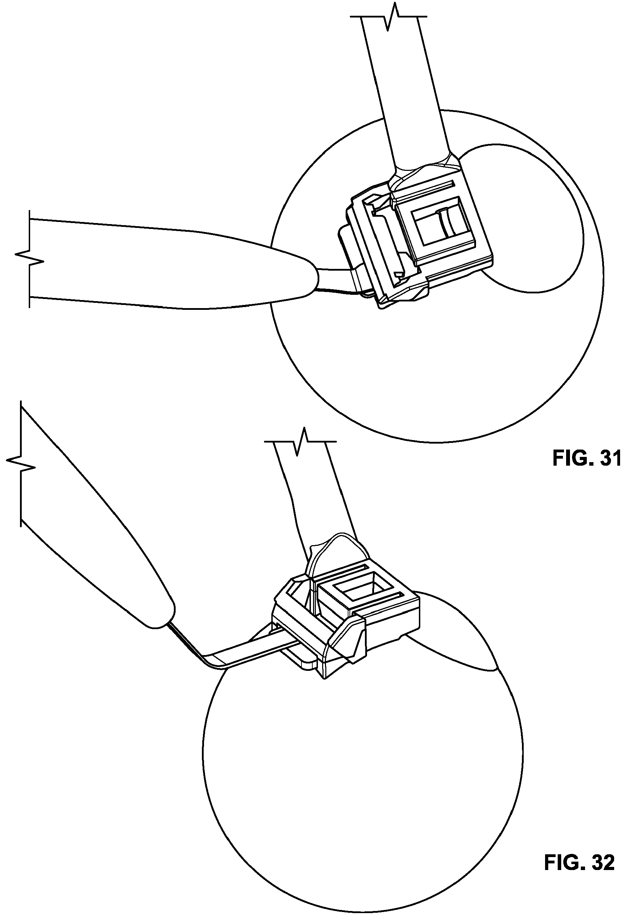

FIGs. 28-32 are illustrations of the use of the kit to make a cut between layers of the sclera. FIG. 28 shows the kit with surgical knife and the guidance device positioned in relation to one another. FIG. 29 shows an alternative perspective of the kit with the surgical knife and the guidance device positioned in relation to one another. FIG. 30 shows the kit with the surgical knife and the guidance device positioned in relation to one another and positioned with respect to the eye. FIG. 31 shows a close-up view of the kit with the surgical knife and the guidance device positioned in relation to one another and positioned with respect to the eye. FIG. 32 shows an alternative perspective close-up view of the kit with the surgical knife and the guidance device positioned in relation to one another and positioned with respect to the eye.

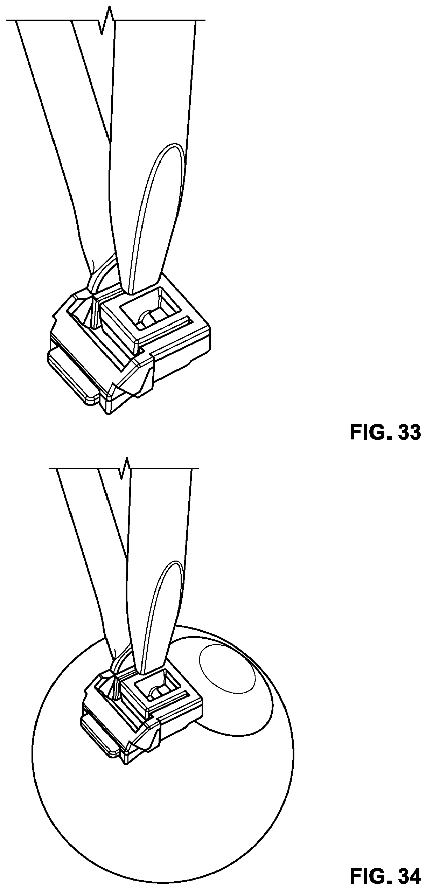

FIGs. 33-34 are illustrations of the use of the kit to make a vertical cut. FIG. 33 shows the surgical knife and the guidance device positioned in relation to one another. FIG. 34 shows the surgical knife and the guidance device positioned in relation to one another and positioned with respect to the eye.

While a number of embodiments of the present disclosure have been described, it is understood that these embodiments are illustrative only, and not restrictive, and that many modifications may become apparent to those of ordinary skill in the art. For example, all dimensions discussed herein are provided as examples only, and are intended to be illustrative and not restrictive.

## Claims

1. A device comprising:
a) a guidance element sized and shaped to be positioned adjacent to a sclera of an eye, the guidance element comprising:
i) a first guide slot configured to guide a first blade in a first direction;
wherein the first guide slot is further configured to allow the first blade to make a first guide cut through a portion of the sclera so as to create an opening to the sclera; and
wherein the first guide slot is further configured to control a depth of entry of the first blade into the first guide slot;
ii) a blade insertion slot configured to receive a second blade;
wherein the blade insertion slot is further configured to allow the second blade to make a first layer cut through the opening of the sclera wherein the first guide cut is present;
wherein the first layer cut is parallel to the sclera; and
wherein the first layer cut extends from the opening of the sclera so as to separate a part of scleral tissue from a remainder of the underlying sclera; and
b) a handle portion.

2. The device of claim 1,
wherein the first blade and the second blade are the same; or wherein the first blade and the second blade are different.

3. The device of claim 1 or 2,
the guidance element further comprising:
iii) a second guide slot configured to guide the first blade in a second direction;
wherein the second guide slot is further configured to allow the first blade to make a second guide cut through a portion of the sclera so as to create a second opening to the sclera; and
wherein the second guide slot is further configured to control a depth of entry of the first blade into the second guide slot;
iv) a third guide slot configured to guide the first blade in a third direction;
wherein the third guide slot is further configured to allow the first blade to make a third guide cut through a portion of the sclera so as to create a second opening to the sclera; and
wherein the third guide slot is further configured to control a depth of entry of the first blade into the third guide slot;
wherein the blade insertion slot is further configured to allow the second blade to make a first, second, and third layer cut through each of the first, second, and third openings defined by the first, second, and third guide cuts respectively;
wherein the first, second, and third layer cuts define a profile of the flap.

4. The device of claim 3,
wherein the profile is a quadrilateral profile.

5. The device of claim 3,
wherein the second and third directions are parallel to each other.

6. The device of claim 5,
wherein the second and third directions are perpendicular to the first direction such that the profile is a rectangular profile.

7. The device of claim 6,
wherein the profile is a square profile.

8. The device of claim 4,
wherein the second and third directions are at an angle of less than 90 degrees relative to the first direction so that the profile is a trapezoidal profile; or wherein the second and third directions are at an angle of more than 90 degrees relative to the first direction so that the profile is a trapezoidal profile.

9. The device of one of claims 1 to 8,
wherein the handle is adjacent to the first guide slot.

10. The device of one of claims 1 to 9,
further comprising one or more anchoring elements configured to attach the guidance elements to a portion of scleral tissue.

11. The device of claim 10,
wherein the one or more anchoring elements comprises a plurality of pins; and/or wherein the one or more anchoring elements comprises a plurality of needles; and/or wherein the one or more anchoring elements comprises a vacuum.

12. The device of one of claims 1 to 11,
further comprising an aiming mechanism, which comprises an arc-shaped portion configured to overlay a limbus line when the guidance device is correctly positioned with respect to the eye.

13. The device of one of claims 1 to 12,
wherein the device is configured to cooperate with a stopper integral with the handle of the blade, wherein the stopper is configured to limit a depth of the first layer cut.

14. A kit comprising:
a) a surgical knife comprising:
i) a first end comprising a first blade; and
ii) a second end comprising a second blade; and
b) A device comprising:
i) a guidance element sized and shaped to be positioned adjacent to a sclera of an eye, the guidance element comprising:
1) a first guide slot configured to guide a first blade in a first direction;
wherein the first guide slot is further configured to allow the first blade to make a first guide cut through a portion of the sclera so as to create an opening to the sclera; and
wherein the first guide slot is further configured to control a depth of entry of the first blade into the first guide slot
2) a blade insertion slot configured to receive a second blade;
wherein the blade insertion slot is further configured to allow the second blade to make a first layer cut through the opening of the sclera wherein the first guide cut is present;
wherein the first layer cut is parallel to the sclera; and wherein the first layer cut extends from the opening of the sclera so as to separate a part of scleral tissue from a remainder of the underlying sclera; and
ii) a handle portion.

15. The kit of claim 14,
wherein the first blade and the second blade are the same; or wherein the first blade and the second blade are different.
